(19) Europäisches Patentamt / European Patent Office / Office européen des brevets

(11) **EP 3 123 914 B1**

(12) **EUROPEAN PATENT SPECIFICATION**

(45) Date of publication and mention
of the grant of the patent:
**03.04.2019 Bulletin 2019/14**

(21) Application number: **15769475.3**

(22) Date of filing: **19.01.2015**

(51) Int Cl.:
*A47K 7/00* (2006.01)   *D21H 27/00* (2006.01)
*D21H 27/30* (2006.01)   *A61Q 19/10* (2006.01)
*A61K 8/02* (2006.01)   *D21H 27/32* (2006.01)
*A47K 10/32* (2006.01)   *A61Q 19/00* (2006.01)

(86) International application number:
**PCT/JP2015/051266**

(87) International publication number:
**WO 2015/146236 (01.10.2015 Gazette 2015/39)**

(54) **WET WIPE AND METHOD FOR MANUFACTURING WET WIPE**

FEUCHTTUCH UND VERFAHREN ZUR HERSTELLUNG DES FEUCHTTUCHS

LINGETTE HUMIDE ET PROCÉDÉ DE FABRICATION DE LINGETTE HUMIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.03.2014 JP 2014070512**

(43) Date of publication of application:
**01.02.2017 Bulletin 2017/05**

(73) Proprietor: **Unicharm Corporation
Ehime 799-0111 (JP)**

(72) Inventors:
• **KONISHI, Takayoshi
Kanonji-shi
Kagawa 769-1602 (JP)**
• **HIRAOKA, Toshio
Kanonji-shi
Kagawa 769-1602 (JP)**

(74) Representative: **Staeger & Sperling
Partnerschaftsgesellschaft mbB
Sonnenstraße 19
80331 München (DE)**

(56) References cited:
WO-A1-2010/021572   JP-A- 2000 290 899
JP-A- 2005 146 451   JP-A- 2006 180 983
JP-A- 2008 002 017   JP-A- 2008 188 071
JP-A- 2008 190 073   JP-A- 2009 155 733
JP-A- 2011 153 387   US-A- 3 881 210
US-A1- 2012 144 611   US-B1- 6 432 095

**Description**

Technical Field

The present disclosure relates to a wet tissue and to a method of producing the wet tissue.

Background Art

Wet tissues of the type that can be flushed in toilets have been developed and are commercially available. For flushing in a toilet, a wet tissue must have a prescribed level of wet strength, considering how it is to be used, and a prescribed level of water disintegratability, considering that it is to be flushed in a toilet. In the relevant technical field, it is common to achieve both wet strength for use and water disintegratability by addition of chemical agents.

For example, PTL 1 describes a water disintegratable sheet containing a water-soluble polymer (methyl cellulose, hydroxypropyl methyl cellulose or the like) and at least one type of compound containing a benzene nucleus substituted with at least two hydroxyl groups (resorcin, pyrocatechol, pyrogallol, phloroglucinol or the like), and a water disintegratable wet tissue.

Also, PTL 2 describes a layered nonwoven fabric having at least two different types of nonwoven fabrics, a water disintegratable nonwoven fabric composed of water disintegratable fibers including ionic fibers formed from a resin composition containing a cationic resin (for example, cationized cellulose, cationized starch, cationized guar gum, cationized dextrin or polydimethylmethylenepiperidinium chloride), and an anionic resin (for example, a polyacrylic acid salt, carboxymethyl cellulose, carboxymethyl starch, alginic acid, xanthan gum or a polymethacrylic acid salt), and a staple fiber nonwoven fabric formed from staple fibers, layered in a water disintegratable manner.

Furthermore, PTL 3 describes a water disintegratable cleaning article comprising a water disintegratable sheet obtained by high-pressure water jet spray treatment of a wet web containing wood pulp, biodegradable synthetic fiber, a water-soluble binder with a carboxyl group and a cationic polymer, which is impregnated with an aqueous cleaning agent containing one or more different metal ions selected from among alkaline earth metals, manganese, zinc, cobalt and nickel, and an organic solvent, the water disintegratable sheet having a multi-ply structure obtained by layering and embossing of two or more monolayer sheets, the monolayer sheet composing the outermost layer being subjected to high-pressure water jet spray treatment from each surface, and being stacked so that the treated surface is facing outward.

In addition, PTL 4 describes a water disintegratable sheet employing an anionic adhesive (carboxymethyl cellulose sodium, carrageenan, sodium polyuronate and the like) and a cationic oligomer represented by formula (1) or (2).

Moreover, as a wet tissue of a type using no chemical agent, PTL 5 describes a water disintegratable fiber sheet including unbeaten pulp (a) with a beating degree of 700 mL or greater, beaten pulp (b) with a beating degree of 400 to 650 mL, regenerated cellulose (c) with a beating degree of 700 mL or greater and refined fibrillated cellulose (d) with a beating degree of 0 to 400 mL.

Citation List

Patent Literature

PTL 1 Japanese Unexamined Patent Publication No. 2001-3297
PTL 2 Japanese Unexamined Patent Publication No. 2001-138424
PTL 3 Japanese Unexamined Patent Publication No. 2008-2017
PTL 4 Japanese Unexamined Patent Publication No. 2009-52152
PTL 5 Japanese Unexamined Patent Publication No. 2010-285718
Further prior art in this technical field is disclosed in documents
JP 2008 002017 A, US 2012/144611 A1, WO 201/021572 A1,
US 6,432,095 B1 and US 3,881,210 A.

Summary of the Invention

Technical Problem

The wet tissues with water disintegratability described in PTL 1 to 4 exhibit both wet strength and water disintegratability by the action of chemical agents. However, considering that a wet tissue is to contact human skin, it is desirable to develop a wet tissue that exhibits both wet strength and water disintegratability without including chemical agents.

Furthermore, while the water disintegratable fiber sheet described in PTL 5 achieves both wet strength and water disintegratability by addition of refined fibrillated cellulose, there is demand for a wet tissue that exhibits both wet strength and water disintegratability by different methods.

It is therefore an object of the present disclosure to provide a wet tissue that exhibits both wet strength and water disintegratability.

Solution to Problem

The inventors have discovered a wet tissue with water disintegratability according to claim 1, including a multilayer sheet comprising a first sheet and a second sheet, wherein each of the first sheet and second sheet includes hydrophilic fibers and hydrophobic fibers.

Advantageous Effects of Invention

The wet tissue of the present disclosure exhibits both wet strength and water disintegratability.

Brief Description of Drawings

Fig. 1 is a plan view of a wet tissue according to one embodiment of the present disclosure.
Fig. 2 is a cross-sectional view along plane II-II of Fig. 1.
Fig. 3 is a schematic diagram for illustration of the relationship between the connected section spacings and the fiber length.
Fig. 4 is a cross-sectional view of a wet tissue according to another embodiment of the present disclosure.
Fig. 5 is a schematic diagram for illustration of a method of producing a wet tissue according to one embodiment of the present disclosure.
Fig. 6 is a schematic diagram for illustration of a method of producing a wet tissue according to one embodiment of the present disclosure. Description of Embodiments

[Definitions]

Several terms will be defined before describing the wet tissue of the present disclosure.

• "Mean fiber length"

As used herein, the mean fiber length of the fibers including the hydrophilic fibers and hydrophobic fibers is the weight-weighted average fiber length, and it is the L(w) value measured using Kajaani fiber Lab fiber properties (off-line)] by Metso Automation.

• "Melting point"

As used herein, the term "melting point" of hydrophobic fibers refers to the peak top temperature for the endothermic peak during conversion from solid to liquid, upon measurement with a differential scanning calorimetry analyzer at a temperature-elevating rate of 10°C/min. The differential scanning calorimetry analyzer used may be, for example, a DSC-60-type DSC measuring apparatus by Shimadzu Corp.

When the hydrophobic fiber includes multiple components, the melting point is measured for each component.

• "Machine direction" and "cross-machine direction"

As used herein, the machine direction is the machine direction during production, and the cross-machine direction is the direction perpendicular to the machine direction during production.

• "Spacing" for connected sections

As used herein, the "spacing" of the connected sections is the distance (inner distance) from one inner side to the other inner side between one connected section and the connected section located nearest to that connected section.

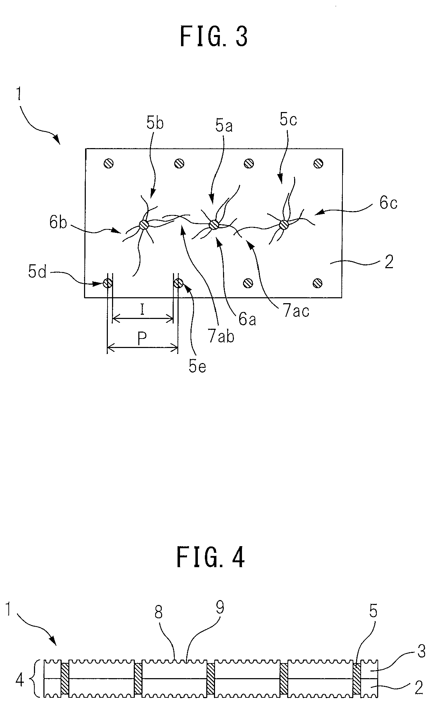

In Fig. 3, the spacing I is the inner distance between a connected section 5d and a connected section 5e located nearest to the connected section 5d.

• "Pitch" for connected sections

As used herein, the "pitch" between the connected sections is the center distance between one connected section and the connected section located nearest to that connected section. In Fig. 3, the pitch P is the center distance between the connected section 5d and the connected section 5e located nearest to the connected section 5d.

The wet tissue of the present disclosure, and a method of producing the wet tissue, will now be explained in detail.

<Wet tissue>

The wet tissue of the present disclosure includes a multilayer sheet with water disintegratability, comprising a first sheet and a second sheet.

As used herein, "wet tissue" and "multilayer sheet" differ in that a "wet tissue" includes a chemical solution while a "multilayer sheet" does not include a chemical solution.

Also, in the drawings for the wet tissue of the present disclosure, the second sheet will sometimes appear to be layered on the first sheet, but this appearance is not intended to restrict the use of the wet tissue. That is, the surface of the first sheet opposite the second sheet is capable of wiping off dirt, while the surface of the second sheet opposite the first sheet is also capable of wiping off dirt.

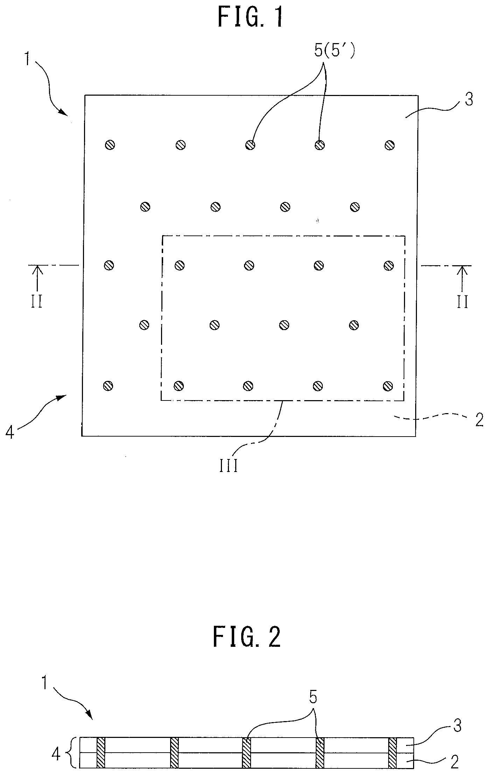

In the wet tissue of the present disclosure, the multilayer sheet has a plurality of connected sections that connect the first sheet and the second sheet, and that are disposed across spacings. Furthermore, the spacings between the plurality of connected sections in the wet tissue of the present disclosure are 0.6 times or above the mean fiber lengths of the hydrophobic fibers in the first sheet and second sheet. Moreover, the plurality of connected sections in the wet tissue of the present disclosure have an area ratio of 0.5 to 12.0% with respect to the multilayer sheet. These will now be explained with reference to Fig. 1 to Fig. 3.

Fig. 1 shows a plan view of a wet tissue according to an embodiment of the present disclosure, and Fig. 2 shows a cross-sectional view along plane II-II of Fig. 1. In Fig. 1 and Fig. 2, the multilayer sheet 4 has a plurality of connected sections 5 that connect the first sheet 2 and the second sheet 3. Specifically, the multilayer sheet 4 in Fig. 1 and Fig. 2 has a plurality of embossed sections 5' formed by embossing the first sheet 2 and the second sheet 3. In the multilayer sheet 4, the plurality of embossed sections 5' are configured in a zigzag fashion.

In the embodiment shown in Fig. 1 and Fig. 2, the connected sections are embossed sections. In a wet tissue according to several other embodiments of the present disclosure which are not part of the present invention, the connected sections are, for example, adhesive sections formed by an adhesive, or pressure-sensitive adhesive sections formed by a pressure-sensitive adhesive.

The connected sections may be formed by connecting the surface of the first sheet and the surface of the second sheet, but preferably the connected sections also have the fibers connected in the interior of either the first sheet or the second sheet, and more preferably they also have the fibers connected in the interiors of both the first sheet and second sheet. This is from the viewpoint of the wet strength of the wet tissue.

From the viewpoint of connecting the fibers together inside either or both the first sheet and second sheet, the connected sections are embossed sections. When the connected sections are embossed sections, the hydrophobic fibers of the first sheet and the second sheet include heat-fusible fibers consisting of composite fibers that include a low-melting-point component and a high-melting-point component having a higher melting point than the low-melting-point component (hereunder also referred to as "heat-fusible fibers consisting of composite fibers that include a low-melting-point component and a high-melting-point component").

In the embossed sections, at least some of the (low-melting-point component of the) heat-fusible fibers of the first sheet are preferably fused with the fibers in the second sheet, and specifically the hydrophilic fiber and/or hydrophobic fibers in the second sheet. Also, in the embossed sections, at least some of the (low-melting-point component of the) heat-fusible fibers of the second sheet are preferably fused with the fibers in the first sheet, and specifically the hydrophilic fiber and/or hydrophobic fibers in the first sheet. This is from the viewpoint of the wet strength of the wet tissue.

If the wet tissue has the aforementioned connected sections, the wet tissue, when wet, will tend to exhibit wet strength that is a combination of the wet strength of the first sheet and the wet strength of the second sheet, and when disintegrated by water, it will tend to exhibit the individual water disintegratability of the first sheet and second sheet.

For example, when the first sheet and second sheet are the same sheet, the wet tissue of the present disclosure will tend to exhibit twice the wet strength of the first sheet, and to exhibit the same water disintegratability as the first sheet.

In the wet tissue of the present disclosure, the spacing between the plurality of connected sections is 0.6 times or above, preferably 0.7 times or above, more preferably 0.8 times or above, even more preferably 1.0 times or above, yet more preferably 1.5 times or above and even yet more preferably 2.0 times or above the mean fiber length of the hydrophobic fibers of the first sheet.

Also in the wet tissue of the present disclosure, the spacing between the plurality of connected sections is 0.6 times or above, preferably 0.7 times or above, more preferably 0.8 times or above, even more preferably 1.0 times or above, yet more preferably 1.5 times or above and even yet more preferably 2.0 times or above the mean fiber length of the hydrophobic fibers of the second sheet.

The reason for which the spacing between the plurality of connected sections should be 0.6 times or above the mean fiber length of the hydrophobic fibers in both the first sheet and second sheet will now be explained with reference to Fig. 3.

Fig. 3 is a schematic diagram for illustration of the relationship between the connected section spacings and the fiber length. Fig. 3 is a plan view of region III of Fig. 1, the second sheet 3 being omitted for ease of explanation. Also in Fig. 3, only the hydrophobic fibers 6a, 6b and 6c connected to the connected sections 5a, 5b and 5c of the first sheet 2 are shown.

If the spacing I between the connected sections 5 is longer than the mean fiber length of the hydrophobic fibers 6 of the first sheet 2, the hydrophobic fibers 6a connected to the connected section 5a, for example, will not connect with the adjacent connected section 5b (or 5c) even if they are tangled with the hydrophobic fibers 6b (or 6c) connected to the adjacent connected section 5b (or 5c) at a tangled point 7ab (or 7ac). In other words, the adjacent connected sections 5a to 5c are not connected by the hydrophobic fibers 6a to 6c. Therefore, when the wet tissue has been discarded in a flush toilet, the connected sections 5a to 5c easily separate into separate fragments so that the water disintegratability of the wet tissue is less likely to be reduced.

The relationship between the spacing between the connected sections and the fiber lengths is also the same for the second sheet.

In a nonwoven fabric such as a wet tissue, the fibers composing the nonwoven fabric generally do not exist in a straight linear form, but are tangled with other fibers and meandering. Thus, even when the spacing I between the connected sections 5 is shorter than the mean fiber length of the hydrophobic fibers 6, the connected sections 5a to 5c will often be separable into different fragments when discarded in a flush toilet or the like.

It has been confirmed by the present inventors that if the spacing of the connected sections has the aforementioned relationship with the hydrophobic fibers in each of the first sheet and second sheet, it is possible to achieve disintegratability of 100 seconds or less in a disintegration test.

Thus, in consideration of water disintegratability, the plurality of connected sections must have a spacing of 0.6 times or above the mean fiber length of the hydrophobic fibers in both the first sheet and second sheet.

For the same reason, the spacing between the plurality of connected sections in the wet tissue of the present disclosure is preferably 0.6 times or above, more preferably 0.7 times or above, even more preferably 0.8 times or above, yet more preferably 1.0 times or above, even yet more preferably 1.5 times or above and most preferably 2.0 times or above the mean fiber lengths of the hydrophilic fibers of the first sheet and second sheet.

Incidentally, since the hydrophilic fibers are connected by hydrogen bonding with the other fibers, and particularly the hydrophilic fibers, the connecting points by hydrogen bonding with the other fibers readily disappear upon disposal in a flush toilet or the like. Thus, the mean fiber length of the hydrophilic fibers has less of an effect on the water disintegratability of the wet tissue than the mean fiber length of synthetic fibers, especially when the connected sections are embossed sections.

In the wet tissue of the present disclosure, the lower limit for the area ratio of the connected sections with respect to the multilayer sheet will differ depending on the area of the individual connected sections, but it is generally 0.5% or greater, preferably 1.0% or greater, more preferably 1.2% or greater and even more preferably 1.5% or greater. If the area ratio is less than 0.5%, connecting the first sheet and the second sheet will be insufficient, resulting in reduced wet strength of the wet tissue and possible tearing of the wet tissue during use.

The upper limit for the area ratio will also differ depending on the area of the individual connected sections and the number density of the connected sections, but it is generally 12% or less, preferably 10.0% or less, more preferably 8.0% or less and even more preferably 5.0% or less. If the area ratio is greater than 12.0%, connecting between the first sheet and the second sheet will be strong and the wet strength will be increased, but when the wet tissue has been discarded in a flush toilet, the first sheet and second sheet will have difficulty separating, the water disintegratability of the wet tissue may be reduced, and the bending resistance of the wet tissue will tend to increase (the wet tissue will become hard).

This upper limit is preferred when the number density of the connected sections is low, such as when the connected sections have a number density of preferably 10 to $1,000/m^2$ and more preferably 50 to $500/m^2$.

As an example where the number density of the connected sections is low, there may be mentioned a working example in which the connected sections are linear connected sections.

When the number density of the connected sections is high, such as when the connected sections have a number density of 1,000 to $100,000/m^2$ and more preferably 10,000 to $70,000/m^2$, the upper limit for the area ratio is preferably 5.0% or less, more preferably 4.5% or less, even more preferably 4.0% or less, and yet more preferably 3.8% or less. As an example where the number density of the connected sections is high, there may be mentioned a working example in which the connected sections are punctiform connected sections.

The area ratio of the connected sections is calculated by the following formula.

$$\text{Area ratio of connected sections (\%)} = 100 \times (\text{total area of connected sections, mm}^2)/(\text{area of multilayer sheet, mm}^2)$$

The number density of the connected sections is the number of connected sections per 1 m$^2$ of the multilayer sheet.

The form of the connected sections is not particularly restricted, and examples of connected sections include punctiform connected sections, for example, connected sections with circular, elliptical, rectangular or triangular shapes, star shapes, heart shapes or any desired character shapes or symbol shapes.

Also, the punctiform connected sections may be disposed on the multilayer sheet without any particular restrictions so long as the spacing is within the prescribed relationship with the mean fiber lengths of the fibers in first sheet and the second sheet, and the punctiform connected sections may be disposed, for example, in an arrangement that is zigzag, such as a square zigzag or 60° zigzag.

The connected sections may be linear connected sections such as straight linear connected sections or non-linear connected sections such as curved connected sections.

Linear connected sections may be arranged, for example, in parallel or non-parallel.

In the wet tissue of the present disclosure, the area per each connected section also varies depending on the area ratio of the connected sections, the shapes of the connected sections, and other factors, but when the connected sections are punctiform connected sections, each of the connected sections has an area of preferably 0.4 to 9.0 mm$^2$, more preferably 0.4 to 7.0 mm$^2$ and even more preferably 1.0 to 5.0 mm$^2$. If the area is less than 0.4 mm$^2$, connecting between the first sheet and the second sheet may be insufficient, and if the area is greater than 9.0 mm$^2$, the wet strength of the wet tissue will tend to be reduced.

Furthermore, when the connected sections are embossed sections, and the area is less than 0.4 mm$^2$, the protrusions on the embossing roll for formation of the embossed sections will be more acute angles which may open holes in the wet tissue, and when the number of embossed sections is increased to increase the wet strength it will become difficult to ensure the spacing between the embossed sections, while if the area is greater than 9.0 mm$^2$, the skin of the user will tend to sense the hardness of the embossed sections.

When the connected sections are linear connected sections, the connected sections have widths of preferably 0.3 to 3.0 mm, more preferably 0.5 to 2.5 mm and even more preferably 1.0 to 2.0 mm. If the widths are less than 0.3 mm, connecting between the first sheet and the second sheet may be insufficient, and the wet strength of the wet tissue may be insufficient. If it exceeds 3.0 mm, the wet strength of the wet tissue will increase but the water disintegratability of the wet tissue will tend to be reduced.

Moreover, when the connected sections are embossed sections, and the widths are less than 0.3 mm, the protrusions of the embossing rolls for formation of the embossed sections will be more acute angles, which may potentially open holes in the wet tissue.

In the wet tissue of this disclosure, each of the first sheet and the second sheet includes hydrophilic fibers and hydrophobic fibers.

As used herein, the simple term "fibers" includes all the fiber types in the first sheet or second sheet.

The hydrophilic fibers are not particularly restricted so long as they are fibers with hydrophilicity and capable of retaining water on the surface or in the interiors. For example, the hydrophilic fibers may be cellulosic fibers, examples of cellulosic fibers including pulp and regenerated cellulose fibers.

Examples of pulp include wood pulp and nonwood pulp. Examples of wood pulp include conifer pulp and broadleaf tree pulp. Examples of nonwood pulp include straw pulp, bagasse pulp, reed pulp, kenaf pulp, mulberry pulp, bamboo pulp, hemp pulp and cotton pulp (such as cotton linter).

Also, the pulp may be non-beaten pulp that has not been subjected to beating treatment, beaten pulp that has been subjected to beating treatment, or a combination thereof.

Non-beaten pulp preferably has a Canadian Standard Freeness of 700 mL or greater.

The Canadian Standard Freeness (CSF) is measured according to JIS P 8121-222012, "Pulp Freeness Test Method - Part 2: Canadian Standard Freeness Method".

The mean fiber length of the non-beaten pulp is not particularly restricted but is generally preferred to be 2 to 4 mm from the viewpoint of economy and productivity.

Beaten pulp is pulp obtained by beating non-beaten pulp by a method such as free beating or wet beating, and it has main body sections and microfiber sections extending from the main body sections. If the wet tissue includes beaten pulp, the wet strength and dry strength of the wet tissue will be increased.

The beaten pulp preferably has a Canadian Standard Freeness of 400 to 650 mL, and more preferably it has a Canadian Standard Freeness of 400 to 600 mL.

The regenerated cellulose fibers may be a rayon such as viscose rayon obtained from viscose, polynosic and modal, or cuprammonium rayon obtained from cuprammonium salt solutions of cellulose, (also known as "cupra"); or lyocell such as Tencel[R], which are not via cellulose derivatives, obtained by organic solvent spinning methods using organic solvents that are mixed solutions of organic compounds and water.

The regenerated cellulose fibers are preferably rayon and especially viscose rayon, from the viewpoint of water absorption, ease of forming the first sheet and second sheet, and economy.

Furthermore, the cellulosic fibers may be, for example, semi-synthetic cellulose fibers such as acetate fibers, among which triacetate fibers and diacetate fibers may be mentioned.

The hydrophobic fibers may be ones commonly used in the technical field, and are preferably synthetic fibers. The synthetic fibers may be ones containing only a single component, such as simple fibers, or ones containing multiple components such as composite fibers.

Examples of the components include polyolefin-based polymers such as polyethylene and polypropylene; polyester-based polymers, for example, terephthalate-based polymers such as polyethylene terephthalate (PET), polybutylene terephthalate and polypentylene terephthalate; polyamide-based polymers such as nylon 6 and nylon 6,6; acrylic polymers; polyacrylonitrile-based polymers; and their modified forms.

When the connected sections are embossed sections, the hydrophobic fibers preferably include heat-fusible fibers, and more preferably they include heat-fusible fibers consisting of composite fibers that include a low-melting-point component and a high-melting-point component having a higher melting point than the low-melting-point component.

In the heat-fusible fibers, the low-melting-point component has a melting point of preferably 120 to 180°C, more preferably 130 to 170°C and even more preferably 140 to 160°C. If the melting point is lower than 120°C, the drying temperature of the first sheet and/or second sheet will need to be lowered to below the melting point of the low-melting-point component in order to prevent fusion of the low-melting-point component, and the productivity of the wet tissue will tend to be reduced.

In the heat-fusible fibers, the high-melting-point component has a melting point of preferably 170 to 300°C, more preferably 180 to 290°C, even more preferably 200 to 270°C and yet more preferably 220 to 260°C. If the melting point is lower than 170°C, not only the low-melting-point component but also the high-melting-point component will undergo melting during the embossing step, and the embossed sections will become hard, sometimes lowering the feel of the wet tissue on the skin. The melting point is preferably not higher than 300°C from the viewpoint of economy.

In the heat-fusible fibers, the low-melting-point component and high-melting-point component have a difference in melting point of preferably 50 to 110°C, more preferably 60 to 100°C and even more preferably 70 to 90°C. If the difference in melting point is less than 50°C, it will tend to be difficult to melt only the low-melting-point component in the embossing step, while if the difference in melting point is greater than 110°C, the melting point of the low-melting-point component will be lower, often resulting in melting of the low-melting-point component during drying of the first sheet and/or second sheet, or the melting point of the high-melting-point component will be high, which is undesirable in terms of economy.

The low-melting-point component and high-melting-point component are not particularly restricted, and may be selected from among the polymers listed for the hydrophobic synthetic fibers.

The low-melting-point component is preferably a terephthalate-based polymer with a lower melting point than PET, and the high-melting-point component is preferably PET.

The composite fibers may be, for example, core-sheath type, core-sheath eccentric type or side-by-side type fibers.

The first sheet includes the hydrophilic fibers and hydrophobic fibers in a proportion of 82 to 95 mass% and 5 to 18 mass%, respectively, more preferably 85 to 95 mass% and 5 to 15 mass%, respectively, even more preferably 88 to 95 mass% and 5 to 12 mass%, respectively, and yet more preferably 90 to 94 mass% and 6 to 10 mass%, respectively, based on the total amount of hydrophilic fibers and hydrophobic fibers.

If the proportion of hydrophobic fibers is less than 5 mass%, the wet strength of the wet tissue will tend to be reduced, and if the proportion of hydrophobic fibers exceeds 18 mass%, the water disintegratability will tend to be reduced.

Furthermore, when the hydrophobic fibers are heat-fusible fibers and the connected sections are embossed sections, and the proportion of hydrophobic fibers is less than 5 mass%, the bonding force between the first sheet and the second sheet by the embossed sections will be reduced and the water disintegratability of the wet tissue will increase, but the wet strength will also be reduced, tending to result in tearing during use, and also tending to reduce the bending resistance (resulting in softness). Moreover, when the hydrophobic fibers are heat-fusible fibers and the connected sections are embossed sections, and the proportion of hydrophobic fibers is greater than 18 mass%, the bonding force between the first sheet and the second sheet by the embossed sections will be increased and the wet strength of the wet tissue will increase, but the water disintegratability will also tend to be inferior and the bending resistance will tend to increase (result in hardness).

In the second sheet, the preferred proportion of the hydrophilic fibers and hydrophobic fibers is the same as in the first sheet.

In the wet tissue of the present disclosure, each of the first sheet and second sheet preferably includes hydrophilic fibers and hydrophobic fibers, and each of the first sheet and second sheet more preferably includes hydrophilic fibers and hydrophobic fibers in the same proportion. This is from the viewpoint of avoiding imbalance in the water disintegratability, wet strength, wiping property, etc. of the wet tissue.

In the wet tissue of the present disclosure, each of the first sheet and second sheet separated from the multilayer sheet exhibits, in a disintegration test as an indicator of water disintegratability, a disintegratability of 100 seconds or less, and preferably exhibits a disintegratability of 90 seconds or less, more preferably 80 seconds or less, and even more preferably 70 seconds or less. If the disintegratability exceeds 100 seconds, toilet pipes etc. may become clogged, depending on their thickness. There is no particular lower limit on the disintegratability.

In Table 1 of "2. Quality" for toilet paper in JIS P 4501:1993 it is stated that toilet paper should satisfy the standard of a disintegratability of no more than 100 seconds, and considering that the wet tissue of the present disclosure is to be discarded in a flush toilet, it preferably has disintegratability equivalent to that of toilet paper.

For the purpose of the present disclosure, the water disintegratability of the first sheet and second sheet separated from the multilayer sheet is used because when the wet tissue is discarded in a flush toilet, usually the water stream strips off the first sheet and second sheet at a relatively early stage, followed by the fragments.

In Table 1 of "2. Quality" for toilet paper in JIS P 4501:1993, it is stated that the aforementioned disintegratability standard is applied for each single sheet when two or more sheets are wound together.

The multilayer sheet is obtained by drying the wet tissue for 24 hours under conditions of 20 $\pm$5°C, 65 $\pm$5% RH, and vaporizing off the chemical solution from the wet tissue.

The wet tissue of the present disclosure preferably also has a disintegratability of 100 seconds or less in a disintegration test for the wet tissue itself, assuming that the first sheet and second sheet will not separate when discarded in a flush toilet, such as when the water stream is weak.

Throughout the present specification, the disintegration test is conducted according to "4.5 Disintegratability" for toilet paper of JIS P 4501:1993. Specifically, it is as follows.

A 300 mL beaker containing 300 mL of water (water temperature: 20°C $\pm$5°C) is placed in a magnetic stirrer, and the rotational speed of the rotor (discoid rotor with diameter: 35 mm, thickness: 12 mm) is adjusted to 600 $\pm$10 rpm. A test strip with 114 $\pm$2 mm sides is loaded into a beaker, and a stopwatch is activated. The rotational speed of the rotor first falls to about 500 rpm due to the resistance of the test strip, the rotational speed increasing as the test strip becomes loose, and upon recovering to 540 rpm, the stopwatch is stopped and the time is measured in second units. The results of disintegratability are expressed as a mean value for 5 tests.

The wet tissue of the present disclosure before impregnation of the chemical solution, i.e. the multilayer sheet, has a bending resistance of 150 mm or less, preferably a bending resistance of 145 mm or less, and more preferably 140 mm or less, and more preferably it has a bending resistance of preferably 135 mm or less. If the bending resistance is greater than 150 mm, the user will tend to feel hardness in the wet tissue. There is no particular lower limit for the bending resistance, but it will generally be 20 mm or above.

As used herein, the bending resistance is measured according to "6.7.3 41.5 Cantilever method" of the general test methods for nonwoven fabrics of JIS L 1913:2010, except that the length of the test strip was changed from "(25 $\pm$1) mm $\times$ (250 $\pm$1) mm" to "(25 $\pm$1) mm $\times$ (200 $\pm$1) mm".

The bending resistance is preferably within the range specified above in any direction of the multilayer sheet, for example, in both the longitudinal and widthwise directions according to the aforementioned JIS standard, such as in both the machine direction and the cross-machine direction during production of the first sheet and the second sheet.

The wet tissue of the present disclosure has a tensile strength of 1.0N or greater and preferably 1.1N or greater per 25 mm width. If the tensile strength is lower than 1.0N per 25 mm width, the wet tissue can potentially tear when the wet tissue is removed.

Throughout the present specification, the tensile strength of the wet tissue may be referred to as the "wet strength" of the wet tissue, and the units of the tensile strength per 25 mm width of the wet tissue may be expressed as "N/25 mm".

The tensile strength is preferably within the range specified above in any direction of the wet tissue, such as in both the machine direction and the cross-machine direction during production of the first sheet and second sheet.

The tensile strength is measured according to "7.1 General method" of the Wet Tensile Strength Test Methods for Paper or Boards" of JIS P 8135:1998, except for the difference specified below.

A multilayer sheet is cut to 25 mm width $\times$ 150 mm length to prepare a sample, which is immersed in distilled water with a mass ratio of 250 mass%, after which the sample is set on a wire mesh for 1 minute. Next, under conditions with an atmosphere of 20°C and 65% relative humidity, the sample is set in a Tensilon tensile tester with a chuck spacing of 100 mm, and the sample is subjected to a tensile test at a pull rate of 100 mm/min, measuring the tensile strength (N) when the sample is torn.

As mentioned above, the multilayer sheet is obtained by drying of the wet tissue for 24 hours under conditions of 20 ±5°C, 65 ±5% RH, and vaporizing off the chemical solution from the wet tissue.

In the wet tissue of the present disclosure, the mean fiber length of the fibers in the first sheet and second sheet, such as the hydrophobic fibers and hydrophilic fibers, is not particularly restricted so long as it satisfies the aforementioned conditions with the spacing of the connected sections formed in the multilayer sheet, but the mean fiber length of the hydrophobic fibers and the mean fiber length of the hydrophilic fibers are preferably 6.5 mm or less, more preferably 6.0 mm or less, and even more preferably 5.5 mm or less. If the mean fiber length is greater than 6.5 mm, the absolute number of tangled points between the fibers in the first sheet and second sheet will increase, tending to lower the water disintegratability.

If the mean fiber length is longer, the absolute number of tangled points between the fibers will increase, thereby tending to lower the water disintegratability and increase the wet strength.

In the wet tissue of the present disclosure, the fibers in the first sheet and second sheet, for example, each of the hydrophobic fibers and hydrophilic fibers (preferably the hydrophilic fibers other than pulp) has a mean fiber length of preferably 2.0 mm or greater, more preferably 2.5 mm or greater and even more preferably 3.0 mm or greater. If the mean fiber length is smaller than 2.0 mm, the wet strength of the first sheet and second sheet will be reduced, and the wet tissue may tear during use.

In the wet tissue of the present disclosure, the multilayer sheet has a basis weight of preferably 20 to 80 $g/m^2$, more preferably 30 to 70 $g/m^2$, even more preferably 40 to 60 $g/m^2$ and yet more preferably 46 to 54 $g/m^2$. If the basis weight is lower than 20 $g/m^2$, the water disintegratability will improve but the wet strength will tend to be reduced, and the bending resistance will tend to be lower. If the basis weight is higher than 80 $g/m^2$, the wet strength will increase but the water disintegratability will tend to be reduced and the bending resistance will tend to be higher.

In the wet tissue of the present disclosure, each of the first sheet and the second sheet, when containing no chemical solution, has a basis weight of preferably 10 to 40 $g/m^2$, more preferably 15 to 35 $g/m^2$, even more preferably 20 to 30 $g/m^2$ and yet more preferably 23 to 27 $g/m^2$. If the basis weight is lower than 10 $g/m^2$, the water disintegratability will improve but the wet strength will tend to be reduced, and the bending resistance will tend to be lower. If the basis weight is higher than 40 $g/m^2$, the wet strength will increase but the water disintegratability will tend to be reduced and the bending resistance will tend to be higher.

In the wet tissue of the present disclosure, the multilayer sheet has a thickness of preferably 0.10 to 0.80 mm, more preferably 0.15 to 0.70 mm, even more preferably 0.20 to 0.60 mm and yet more preferably 0.25 to 0.50 mm. If the thickness is less than 0.10 mm, the water disintegratability of the wet tissue will improve but the wet strength will tend to be reduced, and the bending resistance will tend to be lower. If the thickness is greater than 0.80 mm, the wet strength of the wet tissue will increase but the water disintegratability will tend to be reduced, and the bending resistance will tend to be higher.

The thickness of the multilayer sheet is the thickness in the region of the multilayer sheet where the connected sections are not present.

In the wet tissue of the present disclosure, the first sheet and second sheet have, in the dry state, a thickness of preferably 0.05 to 0.40 mm, more preferably 0.07 to 0.35 mm, even more preferably 0.10 to 0.30 mm and yet more preferably 0.12 to 0.25 mm. If the thickness is less than 0.05 mm, the water disintegratability of the wet tissue will increase but the wet strength will tend to be reduced, and the bending resistance will tend to be lower. If the thickness is greater than 0.40 mm, the wet strength of the wet tissue will increase but the water disintegratability will tend to be reduced, and the bending resistance will tend to be higher.

The thickness of the first sheet and the second sheet is their thickness in the region where the connected sections are not present.

As mentioned above, the multilayer sheet is obtained by drying of the wet tissue for 24 hours under conditions of 20 ±5°C, 65 ±5% RH, and vaporizing off the chemical solution from the wet tissue. Also, the first sheet and second sheet are obtained by detaching the first sheet and second sheet from the multilayer sheet.

The thicknesses of the first sheet and second sheet, and of the multilayer sheet, are measured using an FS-60DS by Daiei Kagaku Seiki Mfg. Co., Ltd., under the conditions, probe: 15 $cm^2$, measuring load: 3 $gf/cm^2$.

In a wet tissue according to another embodiment of the present disclosure, the first sheet has ridges and furrows formed by a high-pressure water jet on the surface opposing the second sheet, and/or the second sheet has ridges and furrows formed by a high-pressure water jet on the surface opposing the first sheet.

Fig. 4 is a diagram illustrating such an embodiment, corresponding to a cross-sectional view along plane II-II of Fig. 1. In the wet tissue 1 shown in Fig. 4, it has a ridge-furrow structure wherein the first sheet 2 has a plurality of ridges 8 and a plurality of furrows 9 formed by a high-pressure water jet on the surface opposing the second sheet 3, and the second sheet 3 includes a plurality of ridges 8 and a plurality of furrows 9 formed by a high-pressure water jet on the surface opposing the first sheet 2. If the wet tissue has a ridge-furrow structure as shown in Fig. 4, the dirt removal property will be improved on both surfaces of the wet tissue.

In the wet tissue of the present disclosure, the pitch of the ridges (furrows) may be freely adjusted by the pitch of the nozzles that spray the high-pressure water jet, but in consideration of ease of formation, and the strength and wiping property of the wet tissue, the pitch of the ridges (furrows) is preferably 0.3 to 1.0 mm.

Also, the heights of the top sections of the ridges and the heights of the bottom sections of the furrows can be adjusted as desired by the pressure of the high-pressure water jet sprayed from the nozzles, but from the viewpoint of the wiping property they are preferably 0.05 to 0.10 mm, more preferably 0.06 to 0.09 mm and even more preferably 0.07 to 0.08 mm.

The steps for forming ridges and furrows in the first sheet and/or second sheet will be explained under "Method of producing wet tissue".

A wet tissue according to yet another embodiment of the present disclosure has a fold structure formed by crepe treatment of the first sheet and/or second sheet. By having a fold structure, the feel of the wet tissue on the skin will improve and the dirt removal property will improve.

The steps for forming a fold structure in the wet tissue will be explained under "Method of producing wet tissue".

For the wet tissue of the present disclosure, chemical solutions with which the multilayer sheet may be impregnated include those used as chemical solutions for wet tissues in the technical field, and are not particularly restricted, with examples including aqueous solutions containing antimicrobial agents, detergents, antiseptic agents and the like, and the chemical solution may even be distilled water.

<Method of producing wet tissue>

The method according to claim 7 of producing the wet tissue of the present disclosure includes the following steps.

(1) A step of forming a first sheet.
(2) A step of forming a second sheet.
(3) A step of layering the second sheet on the first sheet to form a layered sheet, while bonding the layered sheet to form a multilayer sheet having a plurality of connected sections.

The steps of (1) to (3) above will also be referred to as step (1) to step (3), respectively.

Step (1) can be further divided into the following steps.

(1a) A step of supplying an aqueous dispersion of a starting material for the first sheet onto a support, and forming a web of the first sheet on the support.
(1b) A step of spraying the web for the first sheet on the support with a high-pressure water jet from a high-pressure water jet nozzle to tangle the fibers in the web for the first sheet, and form a first sheet.
(1c) A step of drying the first sheet.

The steps of (1a) to (1c) above will also be referred to as step (1a) to step (1c), respectively.

Step (2) can be further divided into the following steps.

(2a) A step of supplying an aqueous dispersion of a starting material for the second sheet onto a support, and forming a web of the second sheet on the support.
(2b) A step of spraying the web for the second sheet on the support with a high-pressure water jet from a high-pressure water jet nozzle to tangle the fibers in the web for the second sheet, and form a second sheet.
(2c) A step of drying the second sheet.

The steps of (2a) to (2c) above will also be referred to as step (2a) to step (2c), respectively.

In step (1a) and step (2a), following a method known in the technical field, the aqueous dispersion of each starting material for the first sheet and second sheet is supplied onto the support, forming the respective webs of the first sheet and second sheet on the support.

In step (1b) and step (2b), the web for the first sheet and the web for the second sheet each receives energy of preferably 0.03 to 0.25 $kW/m^2$, more preferably 0.04 to 0.20 $kW/m^2$, even more preferably 0.05 to 0.15 $kW/m^2$, yet more preferably 0.06 to 0.12 $kW/m^2$ and even yet more preferably 0.07 to 0.10 $kW/m^2$, from the high-pressure water jets discharged from the high-pressure water jet nozzles.

If the energy is less than 0.03 $kW/m^2$, the degree of intertangling of the fibers in the first sheet and second sheet will be insufficient, tending to result in lower wet strength. Moreover if the energy is higher than 0.25 $kW/m^2$, tangling of the fibers of the first sheet and second sheet will progress, increasing the wet strength, but the water disintegratability will tend to be lower, and the bending resistance will tend to be higher.

The high-pressure water jet energy is calculated by the following formula.

$$\text{High-pressure water jet energy (kW/m}^2) = 1.63 \times \text{spray pressure (kg/cm}^2) \times$$
$$\text{spray flow rate (m}^3\text{/min)/transport speed (M/min)/60}$$

The value of the spray flow rate (m³/min) is calculated by the following formula.

$$\text{Spray flow rate (m}^3\text{/min)} = 750 \times \text{orifice total open area (m}^2) \times \text{spray pressure}$$
$$(\text{kg/cm}^2)^{0.495}$$

The spray pressure is the pressure inside the nozzle at the point of spraying from the high-pressure water jet nozzles, the spray flow rate is the total flow per minute of the high-pressure water jet sprayed from the high-pressure water jet nozzles, and the orifice total open area is the total nozzle area of the high-pressure water jet nozzles.

The high-pressure water jet nozzle preferably has hole diameters of 70 to 130 μm. If the hole diameters are smaller than 70 μm the nozzle may tend to become clogged, and if the hole diameters are larger than 130 μm the efficiency of fiber tangling will tend to be reduced.

The high-pressure water jet nozzle pitch will generally be in the range of 0.3 to 1.0 mm.

The high-pressure water jet nozzle sprays the high-pressure water jet onto the web from a distance of preferably 0.5 to 3.0 cm, more preferably 0.5 to 2.0 cm and even more preferably 0.5 to 1.0 cm. If the spacing is less than 0.5 cm the sheet may tear, and if the spacing is greater than 3.0 cm the tangling of fibers in the web will tend to be insufficient.

In step (1c), the first sheet is preferably dried at a temperature that is lower, more preferably a temperature of at least 10°C lower, even more preferably a temperature of at least 20°C lower and yet more preferably a temperature of at least 30°C lower than the melting point of the hydrophobic fibers of the first sheet. If the drying temperature is close to the melting point of the hydrophobic fibers, the hydrophobic fibers may melt during drying, and the hydrophobic fibers may fuse with the other fibers, lowering the water disintegratability of the wet tissue.

When the hydrophobic fibers include multiple components, the melting point is the lowest among the melting points of the multiple components.

When the first sheet includes heat-fusible fibers consisting of composite fibers that include a low-melting-point component and a high-melting-point component, as the hydrophobic fibers, the first sheet is dried in step (1c) at a temperature that is lower, more preferably a temperature of at least 10°C lower, even more preferably a temperature of at least 20°C lower and yet more preferably a temperature that is at least 30°C lower, than the melting point of the low-melting-point component in the first sheet. If the drying temperature is close to the melting point of the low-melting-point component, the low-melting-point component may melt during drying, and the heat-fusible fibers may fuse with the other fibers, lowering the water disintegratability of the wet tissue.

In step (2c), the preferred drying temperature is the same as mentioned for step (1c).

In step (3), using a method known in the technical field, the second sheet is layered on the first sheet to form a layered sheet, and the layered sheet is bonded to form a multilayer sheet having a plurality of connected sections.

In an embodiment in which the connected sections are embossed sections and the hydrophobic fibers are heat-fusible fibers consisting of composite fibers that include a low-melting-point component and a high-melting-point component, the layered sheet is embossed at a temperature of at least the melting point of the low-melting-point component and below the melting point of the high-melting-point component, more preferably the layered sheet is embossed at a temperature of at least 10°C higher than the melting point of the low-melting-point component and more than 10°C below the melting point of the high-melting-point component, even more preferably the layered sheet is embossed at a temperature of at least 20°C higher than the melting point of the low-melting-point component and more than 20°C below the melting point of the high-melting-point component, and even yet more preferably the layered sheet is embossed at a temperature of at least 30°C higher than the melting point of the low-melting-point component and more than 30°C below the melting point of the high-melting-point component.

If the embossing temperature is close to the melting point of the low-melting-point component, melting of the low-melting-point component will be insufficient, and connecting the first sheet and second sheet will also be sufficient, or the time for the embossing step will tend to be longer. If the embossing temperature is close to the melting point of the high-melting-point component, the high-melting-point component will melt and the embossed sections may become hard.

The method of producing the wet tissue of the present disclosure may include, after step (3), the following step: (4) a step of impregnating the multilayer sheet with a chemical solution.

The step of (4) above will also be referred to as step (4).

In step (4), the multilayer sheet is impregnated with a chemical solution by a method known in the technical field.

A method of producing the wet tissue of the present disclosure will now be explained with reference to the drawings.

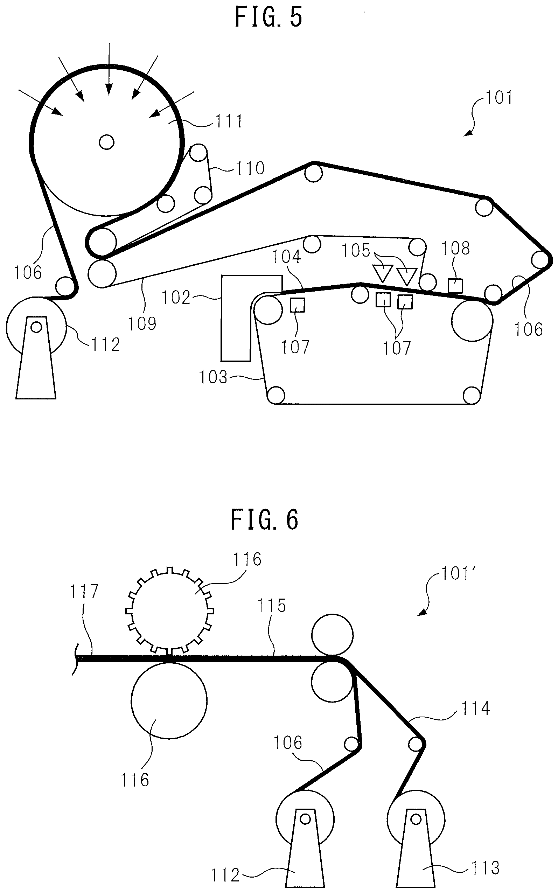

Fig. 5 is a schematic diagram for illustration of a method of producing a wet tissue according to one embodiment of the present disclosure, and specifically of step (1) and step (2).

In the production apparatus 101 shown in Fig. 5, an aqueous dispersion as the starting material for the first sheet is supplied onto a support 103 from a starting material supply head 102, and a web 104 for the first sheet is formed on the support 103.

Next, the web 104 is dewatered with a suction box 107, and the web 104 is passed between two high-pressure water jet nozzles 105 disposed over the support 103, and two suction boxes 107 that collect water sprayed from the high-pressure water jet nozzles 105, disposed at locations facing the high-pressure water jet nozzles 105 in a manner sandwiching the support 103. During passage, the web 104 receives a high-pressure water jet from the high-pressure water jet nozzle 105, tangling the fibers together and forming a first sheet 106 that contains moisture.

Depending on the spacing of the high-pressure water jet nozzles 105, the energy received from the high-pressure water jets, etc., ridges and furrows will sometimes be formed on the surface of the first sheet 106 facing the high-pressure water jet nozzles 105.

Next, the first sheet 106 is transferred to a transport conveyor 109 by a suction pickup 108. The first sheet 106 is then transferred to a transport conveyor 110, after which it is transferred to a dryer 111. The dryer 111 may be a yankee dryer, for example. The dried first sheet 106 is then wound onto a wind-up roll 112.

The second sheet can be produced using the production apparatus 101 shown in Fig. 5, similar to the first sheet, and therefore it will not be explained here. By adjusting the starting material composition and starting material supply rate for production, it is possible to adjust the fiber composition, basis weight, etc. of the second sheet.

Fig. 6 is a schematic diagram for illustration of a method of producing a wet tissue according to one embodiment of the present disclosure, and specifically of step (3) and step (4).

In the production apparatus 101' shown in Fig. 6, the second sheet 114 wound out from the wind-up roll 113 is stacked onto the first sheet 106 wound out from the wind-up roll 112, to form a stacked sheet 115. The stacked sheet 115 is then passed between a pair of embossing rolls 116 that are heated, to form a multilayer sheet 117 having a plurality of embossed sections (not shown).

The multilayer sheet 117 is then cut to a prescribed size and the cut sheet is folded and impregnated with a chemical solution to complete the wet tissue.

In the method of producing a wet tissue according to another embodiment of the present disclosure, in step (3), the second sheet is stacked on the first sheet with the surface of the second sheet that has not been sprayed with the high-pressure water jet facing the surface of the first sheet that has not been sprayed with the high-pressure water jet.

With this embodiment, as shown in Fig. 4, often a wet tissue 1 is formed wherein the first sheet 2 has a plurality of ridges 8 and a plurality of furrows 9 formed by a high-pressure water jet on the surface opposing the second sheet 3, and the second sheet 3 has a plurality of ridges 8 and a plurality of furrows 9 formed by a high-pressure water jet on the surface opposing the first sheet 2. If the wet tissue has ridges and furrows as shown in Fig. 4, the dirt removal property will be improved on both surfaces of the wet tissue.

The method of producing a wet tissue according to yet another embodiment of the present disclosure further includes a step of crepe treatment of the first sheet and/or second sheet. By performing crepe treatment, the wet tissue will have a fold structure, providing effects such as improved dirt removability and improved feel on the skin.

The step of crepe treatment of the first sheet is preferably carried out after step (1c). The step of crepe treatment of the second sheet is also preferably carried out after step (2c).

The crepe treatment is carried out, for example, at the dryer 111 shown in Fig. 5, by pulling the first sheet 106, which is adhered to the surface of the dryer 111, off from the surface using a doctor blade.

The wet tissue of the present disclosure can also be produced by steps known in the prior art, such as a combination of the steps described in Japanese Unexamined Patent Publication No. 2012-202004, Japanese Unexamined Patent Publication No. 2012-20211 and Japanese Unexamined Patent Publication No. 2013-76196, for example.

Examples

The present disclosure will now be explained in fuller detail by examples, with the understanding that it is not meant to be limited to the examples.

[Starting materials]

[Hydrophilic fibers]

•Non-beaten pulp

Northern bleached Kraft pulp (NBKP, CSF: 740 mL) was prepared. • Beaten pulp
The Northern bleached Kraft pulp was mixed with a mixer to obtain beaten pulp having a CSF of 600 mL.

• Rayon (A)

Corona (mean fiber length: 5 mm, 0.7 dtex) by Daiwabo Rayon Co., Ltd. was prepared.

• Rayon (B)

Rayon (mean fiber length: 7 mm, 0.7 dtex) by OmiKenshi Co., Ltd. was prepared.

[Hydrophobic fibers]

•Heat-fusibte fibers

Core-sheath composite fibers (trade name: Tepilus, type: TJ04BN, cut length: 5 mm, 2.2 dtex) by Teijin, Ltd. were prepared. The core was PET with a melting point of 265°C, and the sheath was terephthalate-based fiber with a melting point of 150°C.

[Production Example 1]

Aqueous dispersion No.1 as the starting material for a first sheet was prepared containing 45 parts by mass of beaten pulp, 32 parts by mass of non-beaten pulp, 15 parts by mass of rayon (A) and 8 parts by mass of heat-fusible fibers. In the production apparatus shown in Fig. 5, aqueous dispersion No. 1 as the starting material for the first sheet was supplied onto the support (OS80 by Nippon Filcon Co., Ltd.) from the starting material supply head, and dewatering was carried out from the suction boxes to form web No. 1 for the first sheet.
Next, a high-pressure water jet was sprayed onto web No. 1 for the first sheet from the high-pressure water jet nozzles, while suctioning the water with a suction from below the support, to obtain first sheet No. 1. The high-pressure water jet nozzles were situated at a distance of about 2 cm from above web No. 1 for the first sheet, and they had hole diameters of 92 $\mu$m and hole pitches of 0.5 mm. The energy received by the high-pressure water jets was 0.088 (KW/m$^2$).
Next, first sheet No. 1 was dried for approximately 4 seconds with a yankee dryer kept at 120°C.
Second sheet No. 1 was obtained by the same production method as for first sheet No. 1.
Second sheet No. 1 was stacked onto first sheet No. 1 to form stacked sheet No. 1, and then stacked sheet No. 1 was passed through a pair of embossing rolls that had been heated to 160°C, to form multilayer sheet No. 1 having a plurality of embossed sections. The pair of embossing rolls had rotational axis lines in the direction perpendicular to the machine direction, and had protrusions with diameters of 2.2 mm disposed on the outer peripheral surface of the upper roll in a square zigzag fashion at a pitch of 20 mm in the machine direction and 20 mm in the cross-machine direction, while the surface of the lower roll was flat.
Multilayer sheet No. 1 had embossed sections with diameters of 2.2 mm (area: approximately 3.8 mm$^2$) arranged in a square zigzag fashion at a pitch of 20 mm in the machine direction and 20 mm in the cross-machine direction, the spacing of the embossed sections was approximately 12 mm, and the embossed sections had an area ratio of 1.7% with respect to the multilayer sheet.
Multilayer sheet No. 1 was cut to approximately 20 cm × 13 cm and impregnated with a chemical solution to produce wet tissue No. 1.

[Production Example 2]

First sheet No. 2, second sheet No. 2, multilayer sheet No. 2 and wet tissue No. 2 were produced in the same manner as Production Example 1, except that the upper roll of the pair of embossing rolls was changed to one having protrusions with diameters of 0.88 mm arranged in a 60° zigzag pattern with a pitch of 4.5 mm in the cross-machine direction.
Multilayer sheet No. 2 had embossed sections with diameters of 0.88 mm (area: approximately 0.6 mm$^2$)

arranged in a 60° zigzag fashion at a pitch of 4.5 mm in the cross-machine direction, the spacing of the embossed sections being approximately 3.6 mm, and the embossed sections having an area ratio of 3.4% with respect to the multilayer sheet.

[Production Example 3]

First sheet No. 3, second sheet No. 3, multilayer sheet No. 3 and wet tissue No. 3 were produced in the same manner as Production Example 1, except that the non-beaten pulp was changed to 25 parts by mass, and the heat-fusible fiber was changed to 15 parts by mass.

[Reference Production Example 1]

First sheet No. 4, second sheet No. 4, multilayer sheet No. 4 and wet tissue No. 4 were produced in the same manner as Production Example 1, except that the non-beaten pulp was changed to 40 parts by mass, and the heat-fusible fiber was changed to 0 parts by mass.

[Reference Production Example 2]

First sheet No. 5, second sheet No. 5, multilayer sheet No. 5 and wet tissue No. 5 were produced in the same manner as Production Example 1, except that the upper roll was changed to one having on the outer peripheral surface protrusions with widths of 1.5 mm protruding in the direction perpendicular to the rotational axis line, arranged continuously at a pitch of 10 mm.
Multilayer sheet No. 5 had embossed sections with widths of 1.5 mm, extending in the machine direction, arranged in a striped fashion at a pitch of 10 mm in the cross-machine direction, the spacing between the embossed sections being 8.5 mm, and the embossed sections having an area ratio of 15% with respect to the multilayer sheet.

[Comparative Production Example 1]

A wet tissue was produced according to the method described in PTL 5. Specifically, 26 parts by mass of beaten pulp (CSF: 600 mL), 50 parts by mass of non-beaten pulp (CSF: 740 mL), 21 parts by mass of rayon (B) and 3 parts by mass of fibrillated cellulose fiber were mixed together with water, and a square sheet machine was used to produce a fiber web by a wet paper forming method.
The fiber web was placed on a 100 mesh plastic net, and the fiber web was sprayed with a high-pressure water jet from high-pressure water jet nozzles (nozzle diameter: 92 $\mu$, 0.5 mm pitch) while suctioning off the water by suction from below, after which it was dried with a rotary dryer to obtain sheet No. 6. Sheet No. 6 was impregnated with a chemical solution to obtain wet tissue No. 6. The energy received by the high-pressure water jet was 0.285 (KW/m$^2$).
Incidentally, the fibrillated cellulose fiber was prepared by wet beating Tencel (trade name of Lenzing (Austria), mean fiber length: 3 mm, 1.7 dtex) with a batch macerator (pulper by Aikawa Iron Works Co.) and a continuous macerator (B-type Top Finer by Aikawa Iron Works Co.), and the fiber length in the peak of the weight-weighted average fiber length distribution of the fibrillated cellulose was 3 mm, the mass of the microfiber portion was 1.54 mass%, and the Canadian Standard Freeness was 200 mL.

[Examples 1 to 3, Reference Examples 1 and 2, and Comparative Example 1]

The physical properties of the first sheets, second sheets, multilayer sheets and wet tissues produced in Production Examples 1 to 3, Reference Production Examples 1 and 2 and Comparative Production Example 1 were evaluated.
The results are shown in Table 1.

[Reference Example 3]

The physical properties of first sheet No. 1 produced in Production Example 1 (that is, before formation of the embossed sections) were evaluated. The results are shown in Table 1.
In Table 1, the "basis weight" was calculated by dividing the mass of the sheet by the area.
The "thickness" was measured using an FS-60DS by Daiei Kagaku Seiki Mfg. Co., Ltd. (probe: 15 cm$^2$, measuring load: 3 gf/cm$^2$), and the mean value of the thickness at 3 locations was used.
The "wet strength" for the wet tissue was measured by the method described in the present specification, and for the first sheet and second sheet it was measured in the same manner as for the wet tissue, after allowing the sheet to absorb 250 mass% of distilled water. The tensile strength was measured using an AGS-1kNG autograph by Shimadzu Corp.

The "disintegratability" was measured by the method described in the present specification. For the first sheet and second sheet, the first sheet and second sheet were separated after embossing was formed in the multilayer sheet, and were supplied to a disintegration test. For the wet tissue, it was supplied directly to the disintegration test. The disintegratability was measured using a TTP stirrer for paper disintegration testing, by As One Corp.

The "bending resistance" was measured by the method described in the present specification.

In Table 1, the indication "wet" is for samples measured while containing chemical solution or distilled water, while "dry" is for samples measured without containing chemical solution or distilled water.

Table 1

| Example No. | | Example 1 | | Example 2 | | Example 3 | | Reference Example 1 | | Reference Example 2 | | Reference Example 3 | Comp. Example 1 |
|---|---|---|---|---|---|---|---|---|---|---|---|---|---|
| First sheet and second sheet | | | | | | | | | | | | | |
| Sheet No. | | No. 1 | | No. 2 | | No. 3 | | No. 4 | | No. 5 | | No. 1 | No. 6 |
| First sheet/second sheet | | First | Second | First | Second | First | Second | First | Second | First | Second | First | - |
| Beaten pulp (parts) | | 45 | 45 | 45 | 45 | 45 | 45 | 45 | 45 | 45 | 45 | 45 | 26 |
| Non-beaten pulp (parts) | | 32 | 32 | 32 | 32 | 25 | 25 | 40 | 40 | 32 | 32 | 32 | 50 |
| Rayon (A) (parts) | | 15 | 15 | 15 | 15 | 15 | 15 | 15 | 15 | 15 | 15 | 15 | - |
| Rayon (B) (parts) | | - | - | - | - | - | - | - | - | - | - | - | 21 |
| Heat-fusible fiber (parts) | | 8 | 8 | 8 | 8 | 15 | 15 | - | - | 8 | 8 | 8 | - |
| Fibrillated cellulose fiber (parts) | | - | - | - | - | - | - | - | - | - | - | - | 3 |
| High-pressure water jet energy $(kW/m^2)$ | | 0.088 | 0.088 | 0.088 | 0.088 | 0.088 | 0.088 | 0.088 | 0.088 | 0.088 | 0.088 | 0.088 | 0.285 |
| Dry basis weight $(g/m^2)$ | | 25.3 | 25.3 | 25.3 | 25.3 | 25.1 | 25.1 | 24.9 | 24.9 | 25.3 | 25.3 | 25.0 | - |
| Dry thickness (mm) | | 0.16 | 0.16 | 0.16 | 0.16 | 0.18 | 0.18 | 0.15 | 0.15 | 0.16 | 0.16 | 0.16 | - |
| Wet strength (N/ 25 mm) | Machine direction | 0.9 | | 1.2 | | 1.4 | | 0.7 | | 1.2 | | 0.6 | 1.2 |
| | Cross-machine direction | 0.6 | | 0.9 | | 1.1 | | 0.4 | | 0.7 | | 0.4 | 0.7 |
| Disintegratability | Seconds | 41 | | 73 | | 87 | | 66 | | 104 | | 35 | 104 |

EP 3 123 914 B1

| | | | | | | | |
|---|---|---|---|---|---|---|---|
| Multilayer sheet or wet tissue | | | | | | | |
| Embossed sections | Shape | Round | Round | Round | Round | Striped | - | - |
| | Spacing (mm) | 12 | 3.6 | 12 | 12 | 8.5 | - | - |
| | Area (mm$^2$) | 3.8 | 0.6 | 3.8 | 3.8 | - | - | - |
| | Area ratio (%) | 1.7 | 3.4 | 1.7 | 1.7 | 15 | - | - |
| | Number density (num/m$^2$) | 4,850 | 50,000 | 4,850 | 4,850 | 100 | - | - |
| Embossing spacing/rayon mean fiber length | | 2.40 | 0.72 | 2.40 | 2.40 | 1.70 | - | - |
| Dry basis weight (g/m$^2$) | | 50.6 | 50.6 | 50.2 | 49.8 | 50.6 | - | 50.0 |
| Dry thickness (mm) | | 0.31 | 0.30 | 0.31 | 0.32 | 0.30 | - | 0.30 |
| Wet strength (N/ 25 mm) | Machine direction | 1.6 | 1.9 | 2.2 | 0.9 | 2.0 | - | 3.2 |
| | Cross-machine direction | 1.1 | 1.6 | 1.8 | 0.7 | 1.1 | - | 1.4 |
| Disintegratability | Seconds | 88 | 176 | 227 | 87 | 320 | - | 285 |
| Dry bending resistance (mm) | Machine direction | 134 | 140 | 140 | 125 | 175 | 68 | 135 |
| | Cross-machine direction | 85 | 72 | 90 | 42 | 75 | 42 | 60 |

Table 1 shows that in Examples 1 to 3, the tensile strength of the wet tissue in the machine direction and cross-machine direction was greater than 1.0 N/25 mm, while the disintegratability of the first sheet and second sheet was less than 100 seconds, indicating that both wet strength and water disintegratability had been obtained.

When Example 1 is compared with Reference Example 1 and Reference Example 2, it is seen that formation of embossed sections increased the wet strength but did not significantly affect the water disintegratability.

From Comparative Example 1 it is seen that a higher area ratio of embossed sections lowers the water disintegratability.

Comparison between Examples 1 to 3 and Comparative Example 2 shows that the wet tissues of Examples 1 to 3 have wet strength equivalent to that of Reference Example 1, and higher water disintegratability.

Explanation of Symbols

1 Wet tissue
2 First sheet
3 Second sheet
4 Multilayer sheet
5 Connected section
6 Hydrophobic fiber
7 Tangled point
8 Ridge
9 Furrow
101 Production apparatus
102 Starting material supply head
103 Support
104 Web for first sheet
105 High-pressure water jet nozzle
106 First sheet
107 Suction box
108 Suction pickup
109, 110 Transport conveyors
111 Dryer
112, 113 Wind-up roll
114 Second sheet
115 Stacked sheet
116 Embossing roll
117 Multilayer sheet

**Claims**

1. A wet tissue (1) with water disintegratability, including a multilayer sheet (4) comprising a first sheet (2) and a second sheet:

   wherein each of the first sheet (2) and second sheet (3) includes hydrophilic fibers and hydrophobic fibers (6), the multilayer sheet (4) having a plurality of connected sections (5) connecting the first sheet (2) and the second sheet (3) and being disposed across spacings,
   the spacings between the plurality of connected sections (5) being 0.6 times or above the mean fiber length of the hydrophobic fibers (6) of the hydrophobic fibers of the first sheet (2) and 0.6 times or above the mean fiber length of the hydrophobic fibers (5) of the second sheet (3),
   the plurality of connected sections (5) having an area ratio of 0.5 to 12.0% with respect to the multilayer sheet (4),
   each of the first sheet (2) and second sheet (3), which is separated from the multilayer sheet (4), having a disintegratability of 100 seconds or less in a disintegration test,
   the multilayer sheet (4) having a bending resistance of 150 mm or less, and
   the wet tissue (1) having a tensile strength of 1.0N or greater per 25 mm width,

   wherein each of the first sheet (2) and second sheet (3) includes, as the hydrophobic fibers, heat-fusible fibers consisting of composite fibers that include a low-melting-point component and a high-melting-point component having a higher melting point than the low-melting-point component,

wherein each of the connected sections (5) is an embossed section, and in the embossed section, at least some of the low-melting-point component of the hydrophobic fibers (6) of the first sheet (2) is fused with the fibers in the second sheet (3), and/or at least some of the low-melting-point component of the hydrophobic fibers (6) of the second sheet (3) is fused with the fibers in the first sheet (2),

and wherein each of the first sheet (2) and the second sheet (3) includes the hydrophilic fibers and hydrophobic fibers (6) at a proportion of 82 to 95 mass% and 5 to 18 mass%, respectively, based on total amount thereof.

2. The wet tissue according to claim 1 , wherein the hydrophobic fibers (6) of the first sheet (2) and/or the hydrophobic fibers of the second sheet (3) have a mean fiber length of 6.5 mm or less.

3. The wet tissue according to any one of claims 1 to 2, wherein each of the plurality of embossed sections (5) has an area of 0.4 to 9 mm$^2$.

4. The wet tissue according to any one of claims 1 to 3, wherein in at least the first sheet (2) or the second sheet (3), the hydrophilic fibers (6) include pulp and regenerated cellulose.

5. The wet tissue according to any one of claims 1 to 4, wherein the wet tissue (1) has a disintegratability of 100 seconds or less in a disintegration test.

6. The wet tissue according to any one of claims 1 to 5, wherein the wet tissue (1) has a fold structure formed by crepe treatment of the first sheet (2) and/or second sheet (3).

7. A method of producing the wet tissue according to any one of claims 1 to 6, including the steps of:

   (1) forming a first sheet that includes the steps of:

   (1a) supplying an aqueous dispersion of a starting material for the first sheet onto a support, and forming a web for the first sheet on the support (103),
   (1b) spraying the web for the first sheet (2) on the support (103) with a high-pressure water jet from a high-pressure water jet nozzle (105) to tangle the fibers in the web for the first sheet, and form a first sheet (106), and
   (1c) drying the first sheet (106),

   (2) forming a second sheet that includes the steps of:

   (2a) supplying an aqueous dispersion of a starting material for the second sheet (114) onto a support (103), and forming a web for the second sheet (114) on the support,
   (2b) spraying the web for the second sheet (114) on the support (103) with a high-pressure water jet from a high-pressure water jet nozzle to tangle the fibers in the web for the second sheet (3), and form a second sheet (3), and
   (2c) drying the second sheet (114), and

   (3) stacking the second sheet (114) on the first sheet (106) to form a stacked sheet (115), while bonding the stacked sheet (115) to form a multilayer sheet (117) having a plurality of embossed sections (5), wherein each of the first sheet (106) and the second sheet (114) includes, as hydrophobic fibers, heat-fusible fibers consisting of composite fibers including a low-melting-point component and a high-melting-point component having a higher melting point than the low-melting-point component, in step (1c), the first sheet (106) is dried at a temperature lower than the melting point of the low-melting-point component of the first sheet, and in step (2c), the second sheet (114) is dried at a temperature lower than the melting point of the low-melting-point component of the second sheet (114), and wherein in step (3), the stacked sheet is embossed at a temperature at or above melting points of the low-melting-point component of the first sheet (106) and the low-melting-point component of the second sheet (114), and below melting points of the high-melting-point component of the first sheet (106) and the high-melting-point component of the second sheet (114), to form a multilayer sheet (117) having a plurality of embossed sections.

8. The method according to claim 7, wherein in step (3), the second sheet (3) is stacked on the first sheet (2) with a surface of the second sheet (3) that has not been sprayed with the high-pressure water jet facing a surface of the first sheet (2) that has not been sprayed with the high-pressure water jet, to form a stacked sheet.

9. The method according to any one of claims 7 to 8, further including, after step (1c), a step of crepe treatment of the first sheet, and/or after step (2c), a step of crepe treatment of the second sheet (3).

**Patentansprüche**

1. Feuchttuch (1) mit Wasserzersetzbarkeit, umfassend eine mehrschichtige Lage (4), die eine erste Lage (2) und eine zweite Lage umfasst:

wobei jedes der ersten Lage (2) und zweiten Lage (3) hydrophile Fasern und hydrophobe Fasern (6) umfasst, wobei die mehrschichtige Lage (4) eine Vielzahl von verbundenen Abschnitten (5) aufweist, die die erste Lage (2) und die zweite Lage (3) verbinden und über Abstände angeordnet sind, wobei die Abstände zwischen der Vielzahl der verbundenen Abschnitte (5) 0,6-fach oder höher als die mittlere Faserlänge der hydrophoben Fasern (6) der hydrophoben Fasern der ersten Lage (2) und 0,6-fach oder höher als die mittlere Faserlänge der hydrophoben Fasern (6) der zweiten Lage (3) sind, wobei die Vielzahl der verbundenen Abschnitte (5) ein Flächenverhältnis von 0,5 bis 12,0% in Bezug auf die mehrschichtige Lage (4) aufweist, wobei jede der ersten Lage (2) und der zweiten Lage (3), die von der mehrschichtigen Lage (4) getrennt ist, eine Zersetzbarkeit von 100 Sekunden oder weniger in einem Zersetzungstest aufweist, wobei die mehrschichtige Lage (4) einen Biegesteifigkeit von 150 mm oder weniger aufweist und wobei das Feuchttuch (1) eine Zugfestigkeit von 1,0 N oder mehr pro 25 mm Breite aufweist, wobei jede der ersten Lage (2) und zweiten Lage (3) als hydrophobe Fasern wärmeschmelzbare Fasern umfasst, die aus Verbundfasern bestehen, die eine Niedrigschmelzpunktkomponente und eine Hochschmelzpunktkomponente mit einem höheren Schmelzpunkt als die Niedrigschmelzpunktkomponente umfassen, wobei jeder der verbundenen Abschnitte (5) ein geprägter Abschnitt ist, und in dem geprägten Abschnitt, zumindest ein Teil der Niedrigschmelzpunktkomponente der hydrophoben Fasern (6) der ersten Lage (2) mit den Fasern in der zweiten Lage (3) verschmolzen ist, und/oder zumindest ein Teil der Niedrigschmelzpunktkomponente der hydrophoben Fasern (6) der zweiten Lage (3) mit den Fasern in der ersten Lage (2) verschmolzen ist, und wobei jede der ersten Lage (2) und der zweiten Lage (3) die hydrophilen Fasern und hydrophoben Fasern (6) in einem Anteil von 82 bis 95 Massen-% bzw. 5 bis 18 Massen-%, entsprechend, bezogen auf deren Gesamtmenge, umfasst.

2. Feuchttuch gemäß Anspruch 1, wobei die hydrophoben Fasern (6) der ersten Lage (2) und/oder die hydrophoben Fasern der zweiten Lage (3) eine mittlere Faserlänge von 6,5 mm oder weniger aufweisen.

3. Feuchttuch gemäß einem der Ansprüche 1 bis 2, wobei jeder der Vielzahl von geprägten Abschnitten (5) eine Fläche von 0,4 bis 9 mm$^2$ aufweist.

4. Feuchttuch gemäß einem der Ansprüche 1 bis 3, wobei in zumindest der ersten Lage (2) oder der zweiten Lage (3) die hydrophilen Fasern (6) Zellstoff und regenerierte Zellulose umfassen.

5. Feuchttuch gemäß einem der Ansprüche 1 bis 4, wobei das Feuchttuch (1) eine Zersetzbarkeit von 100 Sekunden oder weniger in einem Zersetzungstest aufweist.

6. Feuchttuch gemäß einem der Ansprüche 1 bis 5, wobei das Feuchttuch (1) eine Faltstruktur aufweist, die durch Kreppbehandlung der ersten Lage (2) und/oder der zweiten Lage (3) ausgebildet ist.

7. Verfahren zur Herstellung des Feuchttuchs gemäß einem der Ansprüche 1 bis 6, das die Schritte umfasst:

(1) Ausbilden einer ersten Lage, das die Schritte umfasst:

(1a) Zuführen einer wässrigen Dispersion eines Ausgangsmaterials für die erste Lage auf einen Träger und Ausbilden eines Gewebes für die erste Lage auf dem Träger (103), (1b) Sprühen des Gewebes für die erste Lage (2) auf den Träger (103) mit einem Hochdruckwasserstrahl aus einer Hochdruckwasserstrahldüse (105), um die Fasern in dem Gewebe für die erste Lage zu verwickeln und eine erste Lage (106) auszubilden, und
(1c) Trocknen der ersten Lage (106),

(2) Ausbilden einer zweiten Lage, das die Schritte umfasst:

(2a) Zuführen einer wässrigen Dispersion eines Ausgangsmaterials für die zweite Lage (114) auf einen Träger (103) und Ausbilden eines Gewebes für die zweite Lage (114) auf dem Träger,
(2b) Sprühen des Gewebes für die zweite Lage (114) auf den Träger (103) mit einem Hochdruckwasserstrahl aus einer Hochdruckwasserstrahldüse, um die Fasern in dem Gewebe für die zweite Lage (3) zu verwickeln und eine zweite Lage (3) auszubilden, und
(2c) Trocknen der zweiten Lage (114), und

(3) Stapeln der zweiten Lage (114) auf der ersten Lage (106), um eine gestapelte Lage (115) auszubilden, während die gestapelte Lage (115) verbunden wird, um eine mehrschichtige Lage (117) mit einer Vielzahl von geprägten Abschnitten (5) auszubilden, wobei jede der ersten Lage (106) und der zweiten Lage (114), als hydrophobe Fasern, Wärmeschmelzfasern, die aus Verbundfasern bestehen, die eine Niedrigschmelzpunktkomponente und eine Hochschmelzpunktkomponente mit einem höheren Schmelzpunkt als die Niedrigschmelzpunktkomponente umfassen, wobei in Schritt (1c) die erste Lage (106) bei einer Temperatur getrocknet wird, die niedriger als der Schmelzpunkt der Niedrigschmelzpunktkomponente der ersten Lage ist, und in Schritt (2c) die zweite Lage (114) bei einer Temperatur getrocknet wird, die niedriger ist als der Schmelzpunkt der Niedrigschmelzpunktkomponente der zweiten Lage (114), und wobei in Schritt (3) die gestapelte Lage bei einer Temperatur bei oder über Schmelzpunkten der Niedrigschmelzpunktkomponente der ersten Lage (106) und der Niedrigschmelzpunktkomponente der zweiten Lage (114) geprägt wird, und unterhalb der Schmelzpunkte der Hochschmelzpunktkomponente der ersten Lage (106) und der Hochschmelzpunktkomponente der zweiten Lage (114), um eine mehrschichtige Lage (117) mit einer Vielzahl von Prägeabschnitten auszubilden.

8. Verfahren gemäß Anspruch 7, wobei in Schritt (3) die zweite Lage (3) auf der ersten Lage (2) mit einer Oberfläche der zweiten Lage (3), die nicht mit dem Hochdruckwasserstrahl besprüht wurde und einer Oberfläche der ersten Lage (2) zugewandt ist, die nicht mit dem Hochdruckwasserstrahl besprüht wurde, gestapelt wird, um eine gestapelte Lage zu bilden.

9. Verfahren gemäß einem der Ansprüche 7 bis 8, das ferner nach Schritt (1c) einen Schritt der Kreppbehandlung der ersten Lage, und/oder nach Schritt (2c), einen Schritt der Kreppbehandlung der zweiten Lage (3) umfasst.

## Revendications

1. Lingette humide (1) avec capacité de désintégration à l'eau, incluant une feuille multicouche (4) comprenant une première feuille (2) et une seconde feuille :

dans laquelle chacune de la première feuille (2) et de la seconde feuille (3) inclut des fibres hydrophiles et des fibres hydrophobes (6),
la feuille multicouche (4) ayant une pluralité de sections raccordées (5) raccordant la première feuille (2) et la seconde feuille (3) et étant disposée à travers des espacements,
les espacements entre la pluralité de sections raccordées (5) étant 0,6 fois ou plus la longueur de fibre moyenne des fibres hydrophobes (6) des fibres hydrophobes de la première feuille (2) et 0,6 fois ou plus la longueur de fibre moyenne des fibres hydrophobes (5) de la seconde feuille (3),
la pluralité de sections raccordées (5) ayant un rapport d'aire de 0,5 à 12,0 % par rapport à la feuille multicouche (4),
chacune de la première feuille (2) et de la seconde feuille (3), qui est séparée de la feuille multicouche (4), ayant une capacité de désintégration de 100 secondes ou moins dans un essai de désintégration,
la feuille multicouche (4) ayant une résistance à la flexion de 150 mm ou moins, et la lingette humide (1) ayant une résistance à la traction de 1,0 N ou plus pour 25 mm de largeur,
dans laquelle chacune de la première feuille (2) et de la seconde feuille (3) inclut, en tant que fibres hydrophobes, des fibres thermofusibles consistant en des fibres composites qui incluent un composant de bas point de fusion et un composant de haut point de fusion ayant un point de fusion plus élevé que le composant de bas point de fusion,
dans laquelle chacune des sections raccordées (5) est une section bosselée, et dans la section bosselée, au moins une partie du composant de bas point de fusion des fibres hydrophobes (6) de la première feuille (2) est fusionnée avec les fibres dans la seconde feuille (3), et/ou au moins une partie du composant de bas point de fusion des fibres hydrophobes (6) de la seconde feuille (3) est fusionnée avec les fibres dans la première feuille (2),

et dans laquelle chacune de la première feuille (2) et de la seconde feuille (3) incluent les fibres hydrophiles et les fibres hydrophobes (6) dans une proportion de 82 à 95 % en masse et 5 à 18 % en masse, respectivement, rapporté à leur quantité totale.

2. Lingette humide selon la revendication 1, dans laquelle les fibres hydrophobes (6) de la première feuille (2) et/ou les fibres hydrophobes de la seconde feuille (3) ont une longueur de fibre moyenne de 6,5 mm ou moins.

3. Lingette humide selon l'une quelconque des revendications 1 à 2, dans laquelle chacune de la pluralité de sections bosselées (5) a une aire de 0,4 à 9 mm$^2$.

4. Lingette humide selon l'une quelconque des revendications 1 à 3, dans laquelle dans au moins la première feuille (2) ou la seconde feuille (3), les fibres hydrophiles (6) incluent de la pâte et de la cellulose régénérée,

5. Lingette humide selon l'une quelconque des revendications 1 à 4, dans laquelle la lingette humide (1) a une capacité de désintégration de 100 secondes ou moins dans un essai de désintégration.

6. Lingette humide selon l'une quelconque des revendications 1 à 5, dans laquelle la lingette humide (1) a une structure de pli formée par un traitement crêpe de la première feuille (2) et/ou de la seconde feuille (3).

7. Procédé de production de la lingette humide selon l'une quelconque des revendications 1 à 6, incluant les étapes de :

(1) formation d'une première feuille qui inclut les étapes de :

(1a) fourniture d'une dispersion aqueuse d'une matière de départ pour la première feuille sur un support, et formation d'un voile pour la première feuille sur le support (103),
(1b) pulvérisation du voile pour la première feuille (2) sur le support (103) avec un jet d'eau haute pression provenant d'une buse de jet d'eau haute pression (105) pour enchevêtrer les fibres dans le voile pour la première feuille, et former une première feuille (106), et
(1c) séchage de la première feuille (106),

(2) formation d'une seconde feuille qui inclut les étapes de :

(2a) fourniture d'une dispersion aqueuse d'une matière de départ pour la seconde feuille (114) sur un support (103), et formation d'un voile pour la seconde feuille (114) sur le support,
(2b) pulvérisation du voile pour la seconde feuille (114) sur le support (103) avec un jet d'eau haute pression provenant d'une buse de jet d'eau haute pression pour enchevêtrer les fibres dans le voile pour la seconde feuille (3), et former une seconde feuille (3), et
(2c) séchage de la seconde feuille (114), et

(3) empilement de la seconde feuille (114) sur la première feuille (106) pour former une feuille empilée (115), tout en liant la feuille empilée (115) pour former une feuille multicouche (117) ayant une pluralité de sections bosselées (5), dans lequel chacune de la première feuille (106) et de la seconde feuille (114) inclut, en tant que fibres hydrophobes, des fibres thermofusibles consistant en des fibres composites incluant un composant à bas point de fusion et un composant à haut point de fusion ayant un point de fusion plus élevé que le composant à bas point de fusion, dans l'étape (1c), la première feuille (106) est séchée à une température plus basse que le point de fusion du composant à bas point de fusion de la première feuille, et dans l'étape (2c), la seconde feuille (114) est séchée à une température plus basse que le point de fusion du composant à bas point de fusion de la seconde feuille (114), et dans lequel dans l'étape (3), la feuille empilée est bosselée à une température à ou au-dessus des points de fusion du composant à bas point de fusion de la première feuille (106) et du composant à bas point de fusion de la seconde feuille (114), et en dessous des points de fusion du composant à haut point de fusion de la première feuille (106) et du composant à haut point de fusion de la seconde feuille (114), pour former une feuille multicouche (117) ayant une pluralité de sections bosselées.

8. Procédé selon la revendication 7, dans lequel dans l'étape (3), la seconde feuille (3) est empilée sur la première feuille (2) avec une surface de la seconde feuille (3) qui n'a pas été pulvérisée avec le jet d'eau haute pression en regard d'une surface de la première feuille (2) qui n'a pas été pulvérisée avec le jet d'eau haute pression, pour former une feuille empilée.

9. Procédé selon l'une quelconque des revendications 7 à 8, incluant en outre, après l'étape (1c), une étape de traitement crêpe de la première feuille, et/ou après l'étape (2c), une étape de traitement crêpe de la seconde feuille (3).

# FIG. 1

# FIG. 2

FIG. 3

FIG. 4

# FIG. 5

# FIG. 6

## REFERENCES CITED IN THE DESCRIPTION

**Patent documents cited in the description**

- JP 2001003297 A
- JP 2001138424 A
- JP 2008002017 A
- JP 2009052152 A
- JP 2010285718 A
- US 2012144611 A1
- WO 201021572 A1
- US 6432095 B1
- US 3881210 A
- JP 2012202004 A
- JP 2012020211 A
- JP 2013076196 A

**Non-patent literature cited in the description**

- Pulp Freeness Test Method - Part 2: Canadian Standard Freeness Method. *JIS P 8121-222012*